# EUROPÄISCHE PATENTSCHRIFT

(11) **EP 0 402 779 B1**
(45) Veröffentlichungstag und Bekanntmachung des Hinweises auf die Patenterteilung: **09.03.2005**
(21) Anmeldenummer: 90110848.0
(22) Anmeldetag: 08.06.1990
(51) Int. Cl.: A61B 5/117, G01N 29/04, G01S 15/89, G01N 29/10

(54) **Verfahren und Vorrichtung zur Ermittlung von Oberflächenstrukturen**
Method and device for determining surface structures
Procédé et dispositif de détermination de structures de surfaces

(30) Priorität: 12.06.1989 DE 3919159; 18.05.1990 DE 4016105
(43) Veröffentlichungstag der Anmeldung: 19.12.1990
(73) Patentinhaber: Sonident Anstalt, 9490 Vaduz (LI)
(72) Erfinder: Grill, Wolfgang, Prof. Dr., D-6240 Königstein (DE)
(74) Vertreter: Funck-Hartherz, Anna-Eleonore, Dipl.-Phys.

(56) Entgegenhaltungen:
- EP-A- 0 048 489
- WO-A-87/05790

## Beschreibung

Die Erfindung bezieht sich auf ein Verfahren und eine Vorrichtung zur Ermittlung von Oberflächenstrukturen und außerdem von oberflächennahen Strukturen von Objekten.

Die in der Optik bekannten Verfahren lassen nur eine Bestimmung der Oberflächenbeschaffenheit zu, jedoch nicht die Bestimmung der nahe unter der Oberfläche befindlichen Strukturen. Diese Tatsache kann zu Fehlbestimmungen führen, da die Oberflächenstruktur leicht äußeren Beeinflussungen, gewollten oder ungewollten, ausgesetzt ist. So sind Identifikationsbestimmungen nach Oberflächenveränderungen nicht mehr durchführbar.

In der EP 0 262 186 ist ein Verfahren und eine Vorrichtung zum Erkennen von Fingerabdrücken und ähnlichen Hautoberflächenstrukturen unter dem Einsatz von Ultraschallwellen beschrieben. Nach dem darin beschriebenen Verfahren wird der Finger oder das jeweilige Objekt auf eine für Ultraschallwellen transparente Auflageplatte gelegt und die zu messenden Bereiche mit einer Frequenz von 10 -15 MHz beschallt. Dabei werden die von der Ultraschallquelle ausgehenden und die von der Objektoberfläche reflektierten Schallwellen durch Flüssigkeit oder einen Festkörper mit entsprechenden Schallausbreitungseigenschaften geleitet. Zur Auswertung wird ein Merkmalsvergleich der reflektierten Schallwellen mit einem einen Fingerabdruck oder ein anderes Oberflächenmuster enthaltendes akustisches Hologramm herangezogen, das sich in einem Aufzeichnungsträger befindet. Der Alternativ-Vorschlag des vorgenannten EP 0 262 286 besteht darin, die reflektierten Schallwellen mittels fokussiertem Schall in geeignete Auswertmerkmale zu transformieren, die dann über einen Empfänger mit einer auf einem Aufzeichnungsträger gespeicherten Merkmalsstruktur verglichen wird.

Zur Erfassung der Oberfläche und oberflächennahen Strukturen wird ausgehend von einer Beschallung des auf einer ebenen Auflagefläche befindlichen Objektes, einer Aufnahme der rückgestreuten Schallwellen von einem Empfänger und einem Schallwellenverlauf durch eine Flüssigkeit oder einen Festkörper erfindungsgemäß vorgeschlagen, dass nur ebene Wellen gesendet und empfangen werden und zwar kontinuierlich oder als Impuls, die Ultraschallwellen so bemessen sind, dass sie etwa gleich oder bis zu 10 mal kürzer sind als die minimale oder maximale Periodenlänge des Ortsftrequenzspektrums des Objektes, eine direkte Messung der zurückgestreuten und reflektierten Wellen ohne Zwischenschaltung von Hilfsmitteln erfolgt und wobei Sender und Empfänger nebeneinander auf einem gemeinsamen Träger in einem Bogen um die Auflage herum bewegbar angeordnet sind. Die Anordnung von Sender und Empfänger kann auch auf die Art und Weise erfolgen, dass diese in konzentrischen Kreisen gegenüber der Auflageplatte angeordnet sind. Infolge der Zusammenwirkung vorgenannter Merkmale wird eine wiederholbare Ermittlung von Oberflächenstrukturen unter Verwendung von Ultraschallwellen erreicht.

Die rückgestreute und reflektierte Welle enthält Informationen über die Oberfläche und die Struktur in der Tiefe von ca. einer Wellenlänge des verwendeten Schalls, da durch die ungleichmäßige Dichte der Struktur die Streuung und Reflexion der Schallwellen beeinflußt wird. Es ist dabei abhängig von der Wellenlänge, welchen Einfluß die Tiefenstruktur hat. Deshalb sind erfindungsgemäß die Wellenlängen der Ultraschallwellen so bemessen, dass sie etwa gleich oder ca. bis zu zehnmal kürzer sind, als die minimale oder maximale Periodenlänge, die sich aus dem Ortsfrequenzspektrum des Objektes ergeben. Das Ortsfrequenzspektrum jeden Objektes beschreibt dieses mittels Frequenzen und errechnet sich mittels der Fourier-Analyse. Die innere Struktur der Oberfläche des zu untersuchenden Objektes ist eine Phasen- und/oder Amplitudenstruktur. Die Phasenstruktur, die eine zeitliche Verschiebung der Welle verursacht, kann eine unsymmetrische Rückstreuung bewirken.

Der Sender der Ultraschallwellen und auch der Empfänger der gestreuten und reflektierten Wellen können getrennt voneinander angeordnet sein; es kann aber auch ein einziger Ultraschallwandler herangezogen werden, da wegen der Reziprozität vieler Umwandlungseffekte sich Ultraschallwandler als Sender und Empfänger verwenden lassen. Es werden solche Ultraschallwandler gewählt, die ebene Wellen aussenden. Als besonders vorteilhaft haben sich scheibenförmige Sender und Empfänger gezeigt. Letztgenannte Sender können aus Piezokeramik sein. Derartige Sender senden parallel zu der Oberfläche des Senders verlaufende Wellenfronten aus, und der Empfänger ist auch nur auf solche Wellen empfindlich, deren Fronten parallel zu dessen Oberfläche verlaufen. Auf diese Weise erhält der Empfänger direkt eine Fouriertransformation, die auf der Fortbewegung der Wellen basiert. Aus der Fouriertransformation kann jeder Parameter wie Phase, Amplitude oder Intensität entnommen werden. Je nach der Struktur des zu untersuchenden Objektes, beispielsweise einer Fingerkuppe, eines Stoffstückes usw., ist die Streuung und Intensität der rückgestreuten Wellen in die verschiedenen Richtungen unterschiedlich stark. Den einzelnen Intensitätswerten können Messeinheiten wie Zahlen, Farben oder andere Kennzeichen zugeordnet werden. Durch eine solche Zuordnung werden bei der Auswertung der rückgestreuten Wellen Bilder erhalten, die leicht vergleichbar sind. Besonders bei der Zuordnung einer Farbskala zu den verschiedenen Intensitätswerten können Bilder erzeugt werden, die eindrucksvoll das untersuchte Objekt wiedergeben.

Der Sender und Empfänger bzw. allgemein der Ultraschallwandler, der Wellen in Signale umwandelt oder umgekehrt, ist beweglich angeordnet und sucht das Objekt nach bestimmten oder allen Winkeln ab. Auf diese Weise gibt es bei jeder Stellung des Wandlers durch die unterschiedliche Streuung und Reflexion eine Information, wobei die Vielzahl der Informationen ein sehr exaktes Bild von der Oberflächenbeschaffenheit und der oberflächennahen Struktur gewährleistet. Selbstverständlich können auch mehrere fest montierte Sender und Empfänger bzw. Ultraschallwandler vorgesehen sein, die mit ebenen Oberflächen ausgestattet sind. Es hat sich gezeigt, dass ein geeignetes Material der Sender, Empfänger oder Wandler Piezokeramik ist.

Der Sender oder Wandler kann entweder kontinuierlich Wellen an das Objekt senden oder nur Wellenimpulse aussenden. Im letzteren Fall ist der Sender von einem Impulsgeber gesteuert.

Gemäß dem erfindungsgemäßen Verfahren kann die Struktur der Oberfläche und die oberflächennahe Struktur untersucht und die Eigenschaften in Form von Informationen abgespeichert werden. Diese Informationen können mit anderen gespeicherten Daten verglichen oder aber mit Hilfe der Methoden der Fourier-Analyse durch Rekonstruktion bildhaft dargestellt werden. Bei dem erfindungsgemäßen Verfahren wird der Sender oder Wandler von einem Frequenzgeber über einen Schalter und gegebenenfalls einen Verstärker angeregt. Für den Fall, dass nur Wellenimpulse auf das Objekt gegeben werden sollen, ist der vorgenannte Schalter impulsgesteuert. Die vom Objekt reflektierten und gestreuten Wellen werden vom Empfänger oder Wandler aufgenommen, der die Information über die Intensität und/oder Phase der Rückstreuung und der Reflexion über einen Detektor, gegebenenfalls auch einen Verstärker sowie eine Zeitsteuerung, die gegebenenfalls mit dem Impulsgeber koordiniert ist, einem Computer zur Analyse und Aufzeichnung eingibt.

Das beschriebene Verfahren eignet sich insbesondere für die Untersuchung von Objekten, wie Fingerkuppen, Dokumenten, Karten usw. Das Verfahren ermöglicht, dass erstmals nicht nur die reine Oberflächenbeschaffenheit, sondern auch die oberflächennahen Strukturen durch das Eindringen der Ultraschallwellen ermittelt werden können, was die Identifikation des untersuchten Materials erheblich verbessert und auch vereinfacht. Bei einem Vergleich mit bereits aufgezeichneten Strukturen und Oberflächenbeschaffenheiten kann man mit wenigen Daten die zu untersuchende Struktur identifizieren und Fälschungen ausschließen. Das führt zum Beispiel bei Ermittlungen der Oberflächenbeschaffenheit und der oberflächennahen Strukturen von Fingerkuppen dazu, dass man die Menge der Daten, die man zum Vergleich braucht, erheblich reduzieren kann, möglicherweise bis auf 30 Bytes. Zusätzlich kann man die Zeit sparen, die für den Vergleich nach herkömmlicher Art benötigt wird. Das erfindungsgemäße Verfahren ist außerdem verschiebungsunabhängig, sofern die Welle eben ist, was die Mühe der Positionierung des Objektes erspart. Lediglich die Drehung der Struktur muß gegebenenfalls kompensiert werden.

Durch die Bestimmung der Dichteunterschiede entsprechend dem erfindungsgemäßen Verfahren wird eine Fälschung der Struktur des zu untersuchenden Objektes sehr erschwert, wenn nicht unmöglich, da Dichteunterschiede der Strukturen erheblich schwieriger zu manipulieren sind als Oberflächenstrukturen.

In den Zeichnungen sind Ausführungsbeispiele von Vorrichtungen zur Durchführung des vorgenannten Verfahren dargestellt. Dabei zeigen:
- Fig. 1: den schematischen Aufbau einer Vorrichtung zur Durchführung des erfindungsgemäßen Verfahrens.
- Fig. 2: ein Schaltschema für die Vorrichtung gemäß Fig. 1,
- Fig. 3: eine modifizierte Ausführung.
- Fig. 4: zeigt eine weitere Modifikation.

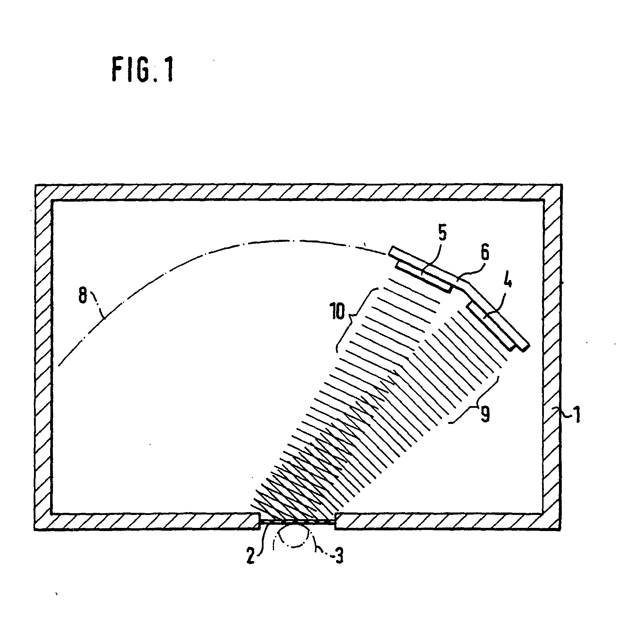

Fig. 1 zeigt eine schematische Darstellung der Vorrichtung zur Durchführung des vorbeschriebenen, zur Ermittelung von Oberflächenstrukturen samt der oberflächennahen Struktur dienenden Verfahrens. Die Vorrichtung besteht aus einem geschlossenen Kasten 1, der mit einem flüssigen Medium, beispielsweise Wasser, gefüllt ist. Die Unterseite des Kastens besitzt eine Auflagefläche 2, gegen die das zu untersuchende und zu bestimmende Objekt angedrückt ist. Die Auflagefläche 2 besteht beispielsweise aus Kunststoff.

Da sich das erfindungsgemäße Verfahren zur Ermittlung von Fingerabdrucken eignet, ist als Objekt in dem Ausführungsbeispiel eine Fingerkuppe 3 dargestellt. Selbstverständlich kann jedes andere Objekt ebenfalls zur Bestimmung herangezogen werden, wie z. B. kristalline Platten, alte Schriften, Textilmaterialien usw. In dem Kasten 1 befindet sich der Sender 4 und der Empfänger 5, die scheibenförmig ausgebildet sind und aus dem gleichen Material, vorzugsweise aus Piezokeramik bestehen. Der Sender und der Empfänger sind an einem gemeinsamen Träger 6 nebeneinander angeordnet, der sich entlang einer Kurve in dem Kasten 1 bewegt. Dazu ist der Träger 6 entsprechend der Bewegungstrajektorie geführt und wird über einen Motor angetrieben. Der Träger 6 ist der zu befahrenen Kurve angepasst. Die Schar der vom Sender 4 ausgesandten parallel verlaufenden Wellenfronten sind mit 9, die zum Empfänger zurückgestreuten Wellenfronten mit 10 bezeichnet. Der Träger bewegt sich auf der Trajektorie 8 und erfasst somit die Fingerkuppe 3 von allen Seiten und ermittelt somit deren Ortsfrequenzspektrum. Ein Fingerabdruck ist mit der Struktur eines Gitters (parallel verlaufende Linien) vergleichbar, da der Abdruck nur aus Linien besteht, die in verschiedenen Richtungen geklumpt verlaufen und bei Gitterstrukturen praktisch nur eine Frequenz anfällt. Eine Fingerkuppe transformiert in ein Frequenzspektrum ergibt eine ziemlich einfache Struktur, die zumindest erheblich einfacher als der Fingerabdruck selbst ist.

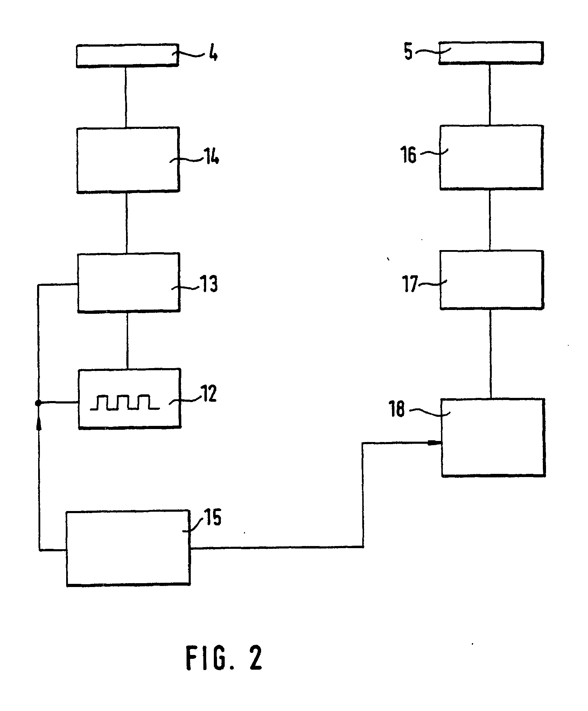

In Fig. 2 ist ein mögliches Schaltschema gezeigt, das die Ultraschallwellen einerseits erzeugt und die rückgestreuten Wellen auswertet sowie eine zeitliche Koordination vornimmt. Ein Ultraschallgenerator 12 speist über einen Schalter 13 und einen Verstärker 14 den Sender 4. Das Steuergerät 15 koordiniert die richtige zeitliche Aufeinanderfolge der Signale. Die von dem Empfänger aufgenommenen Signale werden über einen Verstärker 16, einen Detektor 17 und dem die Auswertung vornehmenden Gerät 18 zugeführt, das mit dem Steuergerät 15 gekoppelt ist. Das Gerät 18 gibt die Digitalinformation der ermittelten Signale an einen Computer weiter.

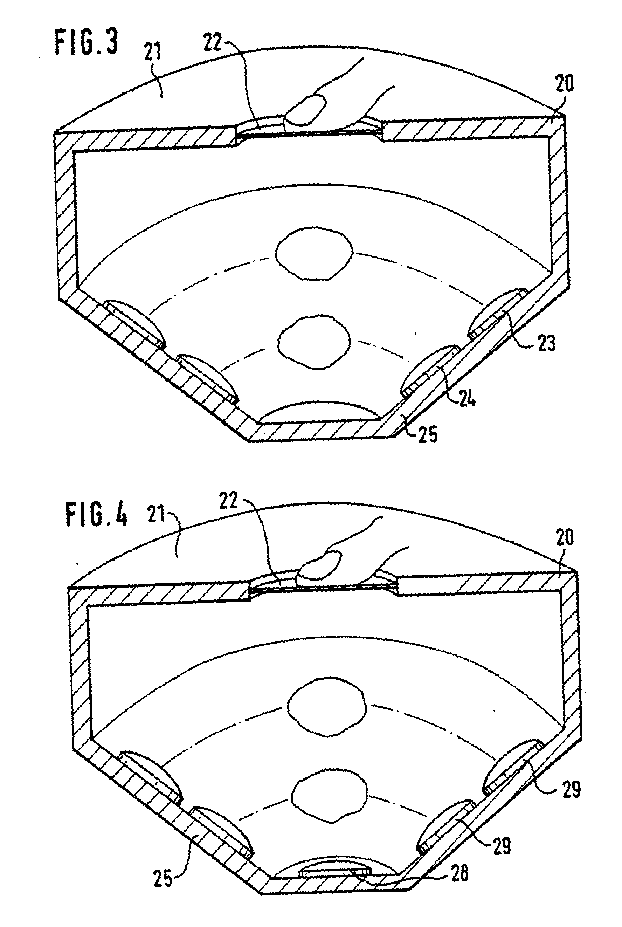

In Fig. 3 ist eine modifizierte Vorrichtung zur Durchführung des erfindungsgemässen Verfahrens gezeigt. Dabei weist der geschlossene Kasten 20 an seiner Oberseite 21 die Auflagefläche 22 auf. Der Kasten 20 besitzt einen kreisrunden Querschnitt und die Sender 23 und die Empfänger 24 sind kreisförmig an schräg zur Mitte hin verlaufenden Bodenteilen 25 angeordnet. Der Kasten 20 kann wahlweise mit einem festen oder flüssigen Medium gefüllt sein. Die Füllung des Kastens mit Flüssigkeit oder einem festen Stoff ist zur Dämpfung der Schallwellen unerlässlich.

Der Sender 23 und der Empfänger 24 sind über das gleiche Schaltschema, wie es in Fig. 2 gezeigt ist, miteinander verbunden. Die einzelnen Sender und Empfänger sind nacheinander geschaltet und zeitlich koordiniert. Die Ausführung gemäß Fig. 4 unterscheidet sich von der Ausführung gemäß Fig. 3 nur dadurch, dass der Sender 28 in der Bodenmitte angeordnet ist und die Empfänger 29 in zwei Kreisen an den geneigten Bodenteilen angeordnet sind.

Mit den vorbeschriebenen Vorrichtungen lassen sich die Oberfläche und die oberflächennahen Strukturen einwandfrei ermitteln. Die jeweilige Wahl der Vorrichtung richtet sich nach dem Objekt und dem Grad der erforderlichen Genauigkeit.

## Patentansprüche

1. Verfahren zur Erfassung von oberflächennahen Strukturen von Objekten mit Hilfe von Ultraschall, wobei die gesamte Fläche des auf einer ebenen Auflageplatte aufliegenden Objektes, z. B. einer Fingerkuppe von Ultraschallwellen beschallt wird und die vom Objekt kommenden Wellen vom Empfänger aufgenommen werden, wobei der gesamte Schallwellenverlauf bis zur Auflagefläche durch Flüssigkeit oder einen Festkörper erfolgt
**dadurch gekennzeichnet,**
**daß** nur ebene Wellen gesendet und empfangen werden und zwar kontinuierlich oder als Impuls,
die Ultraschallwellen so bemessen sind, daß sie etwa gleich oder bis zu 10 mal kürzer sind als die minimale oder maximale Periodenlänge des Ortsfrequenzspektrums des Objektes,
eine direkte Messung der zurückgestreuten und reflektierten Wellen ohne Zwischenschaltung von Hilfsmitteln erfolgt,
und wobei Sender (4) und Empfänger (5) nebeneinander auf einem gemeinsamen Träger (6) in einem Bogen um die Auflageplatte bewegbar angeordnet sind.

2. Verfahren zur Erfassung von oberflächennahen Strukturen von Objekten mit Hilfe von Ultraschall, wobei die gesamte Fläche des auf einer ebenen Auflageplatte aufliegenden Objektes, z. B. einer Fingerkuppe von Ultraschallwellen beschallt wird und die vom Objekt kommenden Wellen vom Empfänger aufgenommen werden, wobei der gesamte Schauwellenverlauf bis zur Auflagefläche durch Flüssigkeit oder einen Festkörper erfolgt
**dadurch gekennzeichnet,**
**daß** nur ebene Wellen gesendet und empfangen werden und zwar kontinierlich oder als Impuls,
die Ultraschallwellen so bemessen sind, daß sie etwa gleich oder bis zu 10 mal kürzer sind als die minimale oder maximale Periodenlänge des Ortsfrequenzspektrums des Objektes,
eine direkte Messung der zurückgestreuten und reflektierten Wellen ohne Zwischenschaltung von Hilfsmitteln erfolgt,
und wobei Sender (23, 28) und Empfänger (24, 29) in konzentrischen Kreisen gegenüber der Auflageplatte (22) angeordnet sind.

## Claims

1. Process to capture the structures close to the surface of objects by means of ultrasonics the whole superficial space of the object lain on the top of a supporting surface being exposed to ultrasound waves, for example a fingertip, and the waves returning from the object being registered by the receiver, the course of all the ultrasound waves until the supporting surface staying the whole way through liquid or solid
**characterized by the fact,**
of being sended or received only flat waves continuously or in impulse,
the length of the ultrasound-waves calculated in this way that they are nearly identical or to ten times shorter than the minimal or maximal periodic length of the spacial frequency spectrum of the object,
of the scattered back or reflected waves being measured without means of any aid
and sender (4) and receiver (5) side by side being installed on a common support (6) in a curve around the supporting surface free movable.

2. Process to capture the structures close to the surface of objects by means of ultrasonics the whole superficial space of the object lain on the top of a supporting surface being exposed to ultrasound waves, for example a fingertip, and the waves returning from the object being registered by the receiver, the course of all the ultrasound waves until the supporting surface staying the whole way through liquid or solid
**characterized by the fact,**
of being sended or received only flat waves continuously or in impulse,
the length of the ultrasound-waves calculated in this way that they are nearly identical or to ten times shorter than the minimal or maximal periodic length of the spacial frequency spectrum of the object,
of the scattered back or reflected waves being measured without means of any aid
and sender (23, 28) and receiver (24, 29) being installed in concentric circles with regard to the supporting surface (22).

## Revendications

1. Procédé technique pour la réquisition des structures des objets près de leurs surface à l'aide d'ultrason, en sonorisant toute la superficie de l'objet posé sur une surface d'appui uni, par exemple un bout du doigt, avec des ondes ultrasonores et les ondes revenant de l'objet enregistrant par le récepteur le cours des ondes ultrasonores passant par liquide ou solide jusqu'à la surface d'appui,
**caracterisé par le fait**
**que** seulement des ondes unies soient emittés et receptés continuellement on par impulsion
**que** la longueur des ondes ultrasonores soie déterminée à peu près semblable ou jusqu'à dix fois plus court comme la durée périodique minimale ou maximale du spectre local de la fréquence de l'objet,
**que** les ondes dispersées ou reflétées soient mesurés sans aide d'autres moyens
et **que** l'emitteur (4) et le récepteur (5) soient monté l'un à coté de l'autre sur un montant commun (6) mobile dans une courbe autour de la surface d'appui.

2. Procédé technique pour la réquisition des structures des objets près de leurs surface à l'aide d'ultrason, en sonorisant toute la superficie de l'objet posé sur une surface d'appui uni, par exemple un bout du doigt, avec des ondes ultrasonores et les ondes revenant de l'objet enregistrant par le récepteur le cours des ondes ultrasonores passant par liquide ou solide jusqu'à la surface d'appui,
**caracterisé par le fait**
**que** seulement des ondes unies soient emitté et recepté continuellement ou par impulsion
**que** la longueur des ondes ultrasonores soie déterminée à peu près semblable ou jusqu'à dix fois plus court comme la durée périodique minimale ou maximale du spectre local de la fréquence de l'objet,
**que** les ondes dispersées ou reflétées soient mesurés sans aide d'autres moyens
et **que** l'emitteur (23,28) et le récepteur (24,29) soient posé en cercles concentriques en regard de la surface d'appui (22).
